(19) Europäisches Patentamt
European Patent Office
Office européen des brevets

(11) EP 0 946 480 B1

(12) EUROPÄISCHE PATENTSCHRIFT

(45) Veröffentlichungstag und Bekanntmachung des Hinweises auf die Patenterteilung:
**07.11.2001 Patentblatt 2001/45**

(21) Anmeldenummer: **97953760.2**

(22) Anmeldetag: **09.12.1997**

(51) Int Cl.[7]: **C07C 45/38**, C07C 45/39, C07C 47/127

(86) Internationale Anmeldenummer:
**PCT/EP97/06856**

(87) Internationale Veröffentlichungsnummer:
**WO 98/28251 (02.07.1998 Gazette 1998/26)**

# (54) VERFAHREN ZUR HERSTELLUNG VON CARBONYLVERBINDUNGEN

METHOD FOR PRODUCING CARBONYL COMPOUNDS

PROCEDE DE PREPARATION DE COMPOSES CARBONYLE

(84) Benannte Vertragsstaaten:
**BE DE ES FR GB IT NL**

(30) Priorität: **23.12.1996 DE 19654054**

(43) Veröffentlichungstag der Anmeldung:
**06.10.1999 Patentblatt 1999/40**

(73) Patentinhaber: **BASF AKTIENGESELLSCHAFT**
**67056 Ludwigshafen (DE)**

(72) Erfinder:
- **FETZER, Thomas**
  **D-67346 Speyer (DE)**
- **DEMUTH, Dirk**
  **D-68161 Mannheim (DE)**
- **RÜTTER, Heinz**
  **D-67126 Hochdorf-Assenheim (DE)**
- **MENIG, Helmuth**
  **D-67159 Friedelsheim (DE)**
- **RESCH, Peter**
  **D-67310 Hettenleidelheim (DE)**
- **RUPPEL, Wilhelm**
  **D-67227 Frankenthal (DE)**
- **WACHE, Harro**
  **D-67136 Fussgönheim (DE)**

(56) Entgegenhaltungen:
**EP-A- 0 007 570** **EP-A- 0 271 812**
**US-A- 4 555 583**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ein neues Verfahren zur Herstellung von Carbonylverbindungen durch Gasphasenoxidation von Alkoholen mit einem Sauerstoff enthaltenden Gas in Gegenwart von Kupfer und/ oder Silber enthaltenden Katalysatoren und einer unter den Reaktionsbedingungen flüchtigen Phosphorverbindung, die direkt in die Katalysatorschüttung eingetragen wird.

Verfahren zur Herstellung von Carbonylverbindungen durch Gasphasenoxidation an Kupfer oder Silber-Katalysatoren in Gegenwart flüchtiger Phosphorverbindungen sind aus dem Stand der Technik bekannt.

So ist in der EP-A 007 570 ein Verfahren zur Herstellung von Glyoxal durch Gasphasenoxidation von Ethylenglykol mit Sauerstoff an einem Kupfer enthaltenden Oxidationskatalysator in Gegenwart von unter Reaktionsbedingungen flüchtigen Phosphorverbindungen beschrieben, bei dem die Phosphormenge von 1 bis 100 ppm, bezogen auf eingesetztes Ethylenglykol, mit den Ausgangsverbindungen zugegeben wird. Bei diesen Verfahren werden unbefriedigende Glyoxalausbeuten bis 70 Mol-%, bezogen auf umgesetztes Ethylenglykol, erzielt.

Nach den Verfahren der US-PS 4 282 374 und der US-PS 4 503 261 erhält man bei der Gasphasenoxidation von Ethylenglykol an Kupferkatalysatoren oder an einem Schichtkatalysator aus Kupfer- und Silberkristallen vorteilhafte Ergebnisse hinsichtlich der Lebensdauer der Katalysatoren und der Glyoxalausbeute, wenn man die Reaktion in Gegenwart einer flüchtigen Phosphorverbindung durchführt, wobei die Phosphormenge (berechnet P), bezogen auf die Gewichtsmenge an Ethylenglykol, 1 bis 100 ppm bzw. 0,5 bis 20 ppm beträgt und mit den Ausgangsverbindungen vor der Katalysatorschüttung zugegeben wird. Bei diesen Verfahren hat sich jedoch bei längerer Betriebsdauer herausgestellt, daß die Glyoxalausbeute und Produktreinheit mit der Versuchsdauer zunehmend schlechter werden. Dieser Nachteil ist auf eine vermehrte Bildung von Formaldehyd und von $CO/CO_2$ zurückzuführen.

In der EP-B 0 271 812 wird für die Herstellung von Carbonylverbindungen wie Glyoxal, eine Gasphasenoxidation von Alkoholen mit einem Sauerstoff enthaltenden Gas in Gegenwart von Kupfer oder Silber enthaltenden Katalysatoren und einer unter den Reaktionsbedingungen flüchtigen Phosphorverbindung vorgeschlagen, bei der die Phosphorverbindung in einer Portion in einer Menge von weniger als 0,5 ppm, bezogen auf die Gewichtsmenge an eingesetztem Alkohol und berechnet als Phosphor, in dem gasförmigen Ausgangsgemisch vor der Umsetzung an dem Katalysator zugemischt wird. Nach dem in der EP-B 0 271 812 beschriebenen Verfahren wird Glyoxal in Ausbeuten bis 80 Mol-% erhalten.

Die vorstehenden Verfahren des Standes der Technik weisen den Nachteil einer unbefriedigenden Ausbeute auf. Bei dem bekannten Verfahren fällt Glyoxal als wäßrige, mit Glykolaldehyd, Formaldehyd und organischen Säuren verunreinigte Lösung an. Weitere unerwünschte Nebenprodukte sind die anfallenden Verbrennungsprodukte CO. $CO_2$ und $H_2O$. Als Folge der Nebenprodukte weisen die bekannten Verfahren zusätzlich den Nachteil unbefriedigender Katalysatorstandzeiten auf.

Anteile von Formaldehyd im Glyoxal sind für viele Anwendungszwecke des Glyoxals zudem wegen der toxikologischen Eigenschaften und der hohen Reaktivität des Formaldehyds höchst unerwünscht. Da man Formaldehyd nur mit einem erheblichen Aufwand und unter Inkaufnahme von Ausbeuteverlusten aus dem rohen Glyoxal entfernen kann, etwa durch Behandlung mit Wasserdampf oder durch chemische Umsetzung, war nach einem Verfahren zu suchen, das es gestattet, Glyoxal durch katalytische Gasphasenoxidation von Ethylenglykol auch bei langen Betriebszeiten unter weitgehender Vermeidung der Bildung störender Nebenprodukte herzustellen.

Es wurde nun gefunden, daß man bei der Herstellung von Carbonylverbindungen der Formel

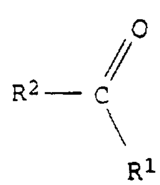

I ,

in der $R^1$ ein Wasserstoffatom oder einen Alkylrest mit 1 bis 3 C-Atomen, $R^2$ ein Wasserstoffatom oder einen Rest der Formel

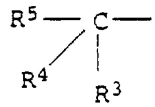 II ,

in der $R^3$ ein Wasserstoffatom oder zusammen mit $R^4$ ein Sauerstoffatom, $R^4$ den Rest $OR^6$ oder zusammen mit $R^3$ ein Sauerstoffatom, $R^5$ ein Wasserstoffatom, einen Alkylrest mit 1 bis 8 C-Atomen oder einen Cyclohexyl- oder Cyclopentylrest und $R^6$ einen Alkylrest mit 1 bis 4 C-Atomen, einen Cyclohexyl- oder Cyclopentylrest oder einen Rest der Formel $-CH_2-CHO$ oder $-CH_2-CH_2-O-CH_2-CHO$ bedeuten, durch Gasphasenoxidation von Methanol oder Alkoholen der Formel

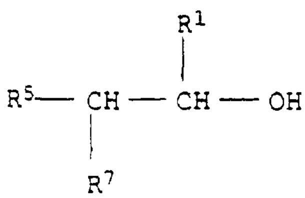 III ,

in der $R^1$ und $R^5$ die oben angegebene Bedeutung haben und $R^7$ ein Wasserstoffatom oder einen Rest $OR^8$ und $R^8$ ein Wasserstoffatom, einen Alkylrest mit 1 bis 4 C-Atomen, einen Cyclohexyl- oder Cyclopentylrest oder einen Rest der Formel $-CH_2-CH_2-OH$ oder $-CH_2-CH_2-O-CH_2-CH_2-OH$ bedeutet, mit einem Sauerstoff enthaltenden Gas in Gegenwart von Kupfer und/oder Silber enthaltenden Katalysatoren und einer unter Reaktionsbedingungen flüchtigen Phosphorverbindung in einer Menge, so daß die Phosphormenge (berechnet als P), bezogen auf die Gewichtsmenge an eingesetztem Alkohol, bis zu 20 ppm, bevorzugt 0,05 bis 20 ppm, beträgt, die Carbonylverbindungen auf besonders vorteilhafte weise herstellen kann, wenn man die Phosphormenge innerhalb der Katalysatorschüttung, bevorzugt im Bereich der oberen 0,1 bis 50 % der Gesamthöhe der Katalysatorschüttung, besonders bevorzugt im Bereich der oberen 1 bis 35 % der Gesamthöhe der Schüttung, zugibt.

Nach dem neuen Verfahren wird Glyoxal aus Ethylenglykol im Dauerbetrieb in hoher Ausbeute und Reinheit und bei deutlich reduziertem Formaldehydanteil erhalten.

In den Alkoholen der Formel III bedeuten Alkylreste z.B. Methyl-, Ethyl-, Propyl- oder Butylreste. Bei dem erfindungsgemäßen Verfahren gehen die endständigen Hydroxylgruppen in Aldehydgruppen und die sekundären Hydroxylgruppen in Ketogruppen über.

Beispielsweise seien die folgende Ausgangsverbindungen der Formel III genannt:

$$HO-CH_2-CH_2-OH, \quad H_3COCH_2-CH_2OH, \quad CH_3-CH_2OH,$$

$$C_2H_5OCH_2-CH_2OH, \quad H_3C-CH(OH)-CH_2-OH,$$

$C_2H_5-CH(OH)-CH_2OH$ , 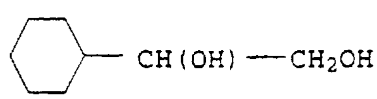 ,

$HO-(CH_2)_2-O-(CH_2)_2-OH$ , 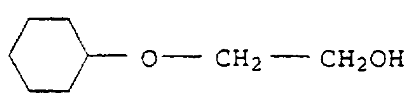 ,

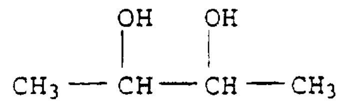

Die Gasphasenoxidation des Alkohols mit dem Sauerstoff enthaltenden Gas an den Kupfer und/oder Silber enthaltenden Katalysatoren führt man auf an sich bekannte Weise, z.B. bei Temperaturen von 225 bis 500°C, durch. Als Kupfer und/oder Silber enthaltende Katalysatoren sind beispielsweise metallisches Kupfer oder Silber, kupferhaltige oder silberhaltige Legierungen oder Verbindungen mit Metallen oder Nichtmetallen geeignet, wie Kupferphosphide, Kupferbronzen oder Legierungen des Kupfers mit Silber und/oder Gold, Kupfererze wie Malachit und Kupfer- oder Silberverbindungen, die während der Reaktion ganz oder teilweise zu Kupfer oder Silber reduziert werden können, wie Kupfer(I)oxid, Silber(I)oxid, Kupfer(II)oxid, und Verbindungen, die beim Erhitzen in Kupferoxide übergehen, wie Kupfernitrat und Kupferacetat. Ferner sind auch Kupferphosphat und Kupferantimonat geeignet. Den kupferhaltigen Verbindungen können zusätzlich noch andere Metall- oder Nichtmetalloxide wie die Oxide von Zink, Chrom, Phosphor, Antimon, Zinn und Wismut beigemischt sein. Die kupfer- und/oder silberhaltige katalytische Masse kann auch auf einem inerten Träger aufgebracht oder gegebenenfalls mit einem inerten Material verdünnt werden. Der Katalysator kann gegebenenfalls auch vor dem Einsatz einer reduzierenden Behandlung unterworfen werden.

Bevorzugt sind Katalysatoren, die keine große innere Oberfläche besitzen, beispielsweise solche mit einer Oberfläche von unter 50 $m^2$ pro g. Besonderes technisches Interesse haben metallisches Kupfer oder Silber und Legierungen, die Kupfer oder Silber als einen wesentlichen Bestandteil enthalten. Man wendet sie z.B. in Form von Drehspänen, Drahtgeweben, Gasen oder auch als Trägerkatalysatoren mit einem z.B. oberflächenarmen, inerten Träger an.

Als unter den Reaktionsbedingungen flüchtige Phosphorverbindungen verwendet man zweckmäßigerweise Phosphorverbindungen, die unzersetzt verdampfbar sind und mit den Komponenten des Synthesegases bei den Umsetzungsbedingungen keine Reaktion eingehen. Das sind z.B. Ester der Phosphorsäure, der phosphorigen Säure oder von Phosphonsäure, wie Trimethylphosphat, Triethylphosphat, Tri-isopropylphosphat, Tri-n-propylphosphat, Trimethylphosphit, Triethylphosphit, Triethylphosphinoxid, Methylphosphonsäurediethylester, Methylphosphonsäuredimethylester oder Ethylphosphonsäurediethylester.

Die Zugabe der Phosphormenge erfolgt nach dem erfindungsgemäßen Verfahren innerhalb der Katalysatorschüttung, bevorzugt im Bereich der oberen 0,1 bis 50 % der Gesamthöhe der Katalysatorschüttung, besonders bevorzugt, im Bereich der oberen 1 bis 35 % der Katalysatorschüttung, nach dem Reaktorkopf. Die Zugabe innerhalb der Katalysatorschüttung erfolgt bevorzugt nach dem Hot Spot. Unter Hot Spot ist der Teil der Katalysatorschüttung zu verstehen, bei dem die höchste Temperatur innerhalb des Temperaturprofils der Katalysatorschüttung auftritt. Das Temperaturprofil der Katalysatorschüttung bzw. die Lage des Hot Spots wird üblicherweise bestimmt, indem man innerhalb der Katalysatorschüttung die Temperatur gegen die Schütthöhe bestimmt. Dies kann beispielsweise durch das Einbringen einer Thermohülse mit einem beweglichen Thermoelement oder aber durch ein feststehendes Multithermoelement mit mehreren Meßpunkten in Abhängigkeit von der Schütthöhe erfolgen.

Die Zugabe der Phosphormenge kann in mehreren, beispielsweise zwei, drei oder vier Anteilen erfolgen, wobei die Zugabe bevorzugt in zwei Anteilen von je 0,05 bis 10 ppm, besonders bevorzugt von je 1 bis 3 ppm, erfolgt.

Bei mehr als zwei Zugabestellen, beispielsweise drei Zugabestellen, wird die Gesamtmenge der flüchtigen Phosphormenge von 20 ppm bevorzugt in Anteile von 0,05 bis 10 ppm aufgeteilt.

Das Gewichtverhältnis des ersten Anteils der Phosphormenge zu dem zweiten beziehungsweise der Summe der weiteren Anteile zugegeben werden, beträgt 0,005 bis 200, vorzugsweise 0,033 bis 30 und besonders bevorzugt 0,3 bis 3,3.

Das erfindungsgemäße Verfahren wird z.B. so ausgeführt, daß man ein gasförmiges Gemisch aus dem Alkohol und Wasser, wobei der Wassergehalt 0,1 bis 99 Gew.-% beträgt, zusammen mit Luft oder Sauerstoff in einer Menge zwischen 0,5 und 2,0 mol, bezogen auf 1 mol eingesetzten Alkohols, eventuell zusammen mit Stickstoff in einer Menge von bis zu 99 Vol.-% des Gesamtgasgemisches, über den auf 225 bis 500°C gehaltenen Katalysator leitet, wobei die flüchtige Phosphorverbindung in die Katalysatorschüttung nach dem Hot Spot im Bereich von 1 bis 35 % der Gesamthöhe der Schüttung zugibt.

Das den Reaktor verlassende Gasgemisch wird üblicherweise mit Wasser ausgewaschen.

Man kann die Phosphorverbindung als Lösung in Wasser, Alkohol, bevorzugt dem eingesetzten Alkohol, oder geeigneten Lösungsmitteln, wie zum Beispiel Ethern, in flüssiger oder durch Verdampfen der Lösung in gasförmiger Form oder aber in Form der reinen gasförmigen Phosphorverbindung zugeben, wobei die Zugabe als verdampfte Lösung oder in reiner gasförmiger Form bevorzugt ist.

Das nach dem erfindungsgemäßen Verfahren aus Ethylenglykol erhaltene Glyoxal, das direkt in der handelsüblichen Form einer 40 gew.-%igen wässrigen Lösung erhalten werden kann, zeichnet sich durch eine hohe Reinheit aus, die auch bei langem Betriebszeitraum unverändert erhalten bleibt. Durch das erfindungsgemäße Verfahren wird Glyoxal in hohen Ausbeuten bei langen Katalysatorstandzeiten erhalten.

Das erfindungsgemäße Verfahren wird anhand der nachfolgenden Beispiele näher erläutert.

Beispiel 1

7,2 kg Kupferformkörper wurden in einem Rohrreaktor aus rostfreiem Stahl mit einem inneren Durchmesser von 55 mm so eingebaut, daß die Katalysatorfüllhöhe 250 cm beträgt (Katalysatorvolumen: 5,7 1). Man leitete pro Stunde ein Synthesegasgemisch bestehend aus 840 g Ethylenglykol, 1720 N1 Luft und 230 N1 Stickstoff durch den Rohrreaktor. Zum Synthesegas wurden bei 24 % der Gesamthöhe der Katalysatorschüttung nach dem Hot Spot, der bei 15 % der Gesamthöhe der Schüttung lag, 0,3 ppm P, bezogen auf die eingesetzte Gewichtsmenge an Ethylenglykol, in Form von Triethylphosphat gegeben. Die Reaktortemperatur wurde mit Hilfe einer Salzschmelze auf 365°C eingestellt.

Die Gesamtgasmenge, bestehend aus Kreisgas und Synthesegas, betrug 9150 N1/h. Die GHSV (gas hourly space velocity), die definiert ist als

$$GHSV = Gasvolumen / Katalysatorvolumen$$

betrug 1610 $h^{-1}$. Die LHSV (liquid hourly space velocity), die definiert ist als

$$LHSV = Flüssigkeitsvolumen / Katalysatorvolumen$$

betrug 0,13 $h^{-1}$. Die Verweilzeit, definiert als Quotient aus dem Katalysatorvolumen und der Gasmenge war 2,3 s.

Das Reaktionsgas wurde nach Verlassen des Reaktors mit Wasser in Berührung gebracht, wobei die Reaktionsprodukte in der wässrigen Phase gelöst wurden. Die bei der Reaktion entstandenen Permanentgase CO und $CO_2$ verblieben im Abgas und wurden in der Gasphase analysiert.

Nach einer Laufzeit von 10 Tagen erhielt man eine Glyoxalausbeute von 76,9 Mol-%, bezogen auf eingesetztes Ethylenglykol, bei einem Ethylenglykolumsatz von 98,0 Mol-%. Die Verbrennung zu CO und $CO_2$ betrug 12,9 Mol-%. Als weitere Nebenprodukte entstanden 1,7 Mol-% Glyoxaldehyd und 4,9 Mol-% Formaldehyd.

Vergleichsbeispiel 1 (kein Zusatz flüchtiger P-Verbindung)

Man verfuhr wie in Beispiel 1 beschrieben. Es wurde jedoch keine flüchtige Phosphorverbindung zugesetzt. Die Produktaufarbeitung und die Gasanalytik erfolgte wie in Beispiel 1 beschrieben. Man erhielt eine Glyoxal-Ausbeute von 71,6 Mol-% bei einem Ethylenglykolumsatz von 94,3 Mol-%. Die Verbrennug zu CO und $CO_2$ betrug 13 Mol-%. Glykolaldehyd und Formaldehyd wurden 1,3 Mol-% und 6,1 Mol-% gebildet.

Vergleichsbeispiel 2 (Zusatz flüchtiger P-Verbindungen zum Ausgangsgemisch)

Man verfuhr wie in Beispiel 1 beschrieben, wobei jedoch die Zugabe von Triethylphosphat vor der Katalysatorschüttung erfolgte. Dem Synthesegas wurden vor der Katalysatorschüttung 0,3 ppm P, bezogen auf die eingesetzte Gewichtsmenge an Ethylenglykol, in Form von Triethylphosphat zugegeben. Die Produktaufarbeitung und die Gasanalytik erfolgte wie in Beispiel 1 beschrieben. Man erhielt eine Glyoxalausbeute von 75,9 Mol-% bei einem Ethylenglykolumsatz von 98,8 Mol-%. Die Verbrennung zu CO und $CO_2$ betrug 13,8 Mol-%. Glykolaldehyd und Formaldehyd wurden 1,1 Mol-% und 5,9 Mol-% gebildet.

Die folgende Tabelle 1 stellt die mit den erfindungsgemäßen Beispiel 1 den mit den nicht erfindungsgemäßen Vergleichsbeispielen V1 und V2 erzielten Umsätzen und Ausbeuten gegenüber.

Tabelle 1

| Beispiel | Ethylenglykol-Umsatz [Mol-%] | Glyoxal-Ausbeute [Mol-%] | $CO/CO_2$ Ausbeute [Mol-%] | Glykolaldehyd Ausbeute [Mol%] | Formaldehyd Ausbeute [Mol-%] |
|---|---|---|---|---|---|
| 1 | 98,0 | 76,9 | 12,9 | 1,6 | 4,9 |

Tabelle 1 (fortgesetzt)

| Beispiel | Ethylenglykol-Umsatz [Mol-%] | Glyoxal-Ausbeute [Mol-%] | $CO/CO_2$ Ausbeute [Mol-%] | Glykolaldehyd Ausbeute [Mol%] | Formaldehyd Ausbeute [Mol-%] |
|---|---|---|---|---|---|
| V1 | 94,3 | 71,6 | 13 | 1,3 | 6,1 |
| V2 | 98,8 | 75,9 | 13,8 | 1,1 | 5,9 |

Beispiel 2

7,2 kg Kupferformkörper wurden in einem Rohrreaktor aus rostfreiem Stahl mit einem inneren Durchmesser von 55 mm so eingebaut, daß die Katalysatorfüllhöhe 250 cm beträgt (Katalysatorvolumen: 5,7 1). Man leitete pro Stunde ein Synthesegasgemisch bestehend aus 840 g Ethylenglykol, 1800 N1 Luft und 150 N1 Stickstoff durch den Rohrreaktor. Zum Synthesegas wurden bei 24 % der Gesamthöhe der Katalysatorschüttung nach dem Hot Spot, der bei 15 % der Gesamthöhe der Schüttung lag, 0,3 ppm P, bezogen auf die eingesetzte Gewichtsmenge an Ethylenglykol, in Form von Triethylphosphat gegeben. Die Reaktortemperatur wurde mit Hilfe einer Salzschmelze auf 365°C eingestellt.

Die Gesamtgasmenge, bestehend aus Kreisgas und Synthesegas, betrug 9150 N1/h. Die GHSV (gas hourly space velocity), die definiert ist als

GHSV = Gasvolumen / Katalysatorvolumen

betrug 1610 $h^{-1}$. Die LHSV (liquid hourly space velocity), die definiert ist als

LHSV = Flüssigkeitsvolumen / Katalysatorvolumen

betrug 0,13 $h^{-1}$. Die Verweilzeit, definiert als Quotient aus dem Katalysatorvolumen und der Gasmenge war 2,3 s.

Das Reaktionsgas wurde nach Verlassen des Reaktors mit Wasser in Berührung gebracht, wobei die Reaktionsprodukte in der wässrigen Phase gelöst wurden. Die bei der Reaktion entstandenen Permanentgase CO und $CO_2$ verblieben im Abgas und wurden in der Gasphase analysiert.

Nach einer Laufzeit von 10 Tagen erhielt man eine Glyoxalausbeute von 76,0 Mol-%, bezogen auf eingesetztes Ethylenglykol, bei einem Ethylenglykolumsatz von 98,7 Mol-%. Die Verbrennung zu CO und $CO_2$ betrug 14,4 Mol-%. Als weitere Nebenprodukte entstanden 1,1 Mol-% Glyoxaldehyd und 4,7 Mol-% Formaldehyd.

Vergleichsbeispiel 3 (kein Zusatz flüchtiger P-Verbindung)

Man verfuhr wie in Beispiel 2 beschrieben. Es wurde jedoch keine flüchtige Phosphorverbindung zugesetzt. Die Produktaufarbeitung und die Gasanalytik erfolgte wie in Beispiel 2 beschrieben. Man erhielt eine Glyoxal-Ausbeute von 73,8 Mol-% bei einem Ethylenglykolumsatz von 98,2 Mol-%. Die Verbrennug zu CO und $CO_2$ betrug 14,9 Mol-%. Glykolaldehyd und Formaldehyd wurden 0,8 Mol-% und 5,8 Mol-% gebildet.

Vergleichsbeispiel 4 (Zusatz flüchtiger P-Verbindungen zum Ausgangsgemisch)

Man verfuhr wie in Beispiel 2 beschrieben, wobei jedoch die Zugabe von Triethylphosphat vor der Katalysatorschüttung erfolgte. Dem Synthesegas wurden vor der Katalysatorschüttung 0,3 ppm P, bezogen auf die eingesetzte Gewichtsmenge an Ethylenglykol, in Form von Triethylphosphat zugegeben. Die Produktaufarbeitung und die Gasanalytik erfolgte wie in Beispiel 2 beschrieben. Man erhielt eine Glyoxalausbeute von 74,5 Mol-% bei einem Ethylenglykolumsatz von 99,6 Mol-%. Die Verbrennung zu CO und $CO_2$ betrug 16 Mol-%. Glykolaldehyd und Formaldehyd wurden 0,5 Mol-% und 3,3 Mol-% gebildet.

Die folgende Tabelle 2 stellt die mit den erfindungsgemäßen Beispiel 2 den mit den nicht-erfindungsgemäßen Vergleichsbeispielen V3 und V4 erzielten Umsätzen und Ausbeuten gegenüber.

Tabelle 2

| Beispiel | Ethylenglykol-Umsatz [Mol-%] | Glyoxal-Ausbeute [Mol-%] | $CO/CO_2$ Ausbeute [Mol-%] | Glykolaldehyd Ausbeute [Mol%] | Formaldehyd Ausbeute [Mol-%] |
|---|---|---|---|---|---|
| 2 | 98,7 | 76,0 | 14,4 | 1,1 | 4,7 |
| V3 | 98,2 | 73,8 | 14,9 | 0,8 | 5,8 |
| V4 | 99,6 | 74,5 | 16 | 0,5 | 3,3 |

Beispiel 3

7,2 kg Kupferformkörper wurden in einem Rohrreaktor aus rostfreiem Stahl mit einem inneren Durchmesser von 55 mm so eingebaut, daß die Katalysatorfüllhöhe 250 cm beträgt (Katalysatorvolumen: 5,7 1). Man leitete pro Stunde ein Synthesegasgemisch bestehend aus 840 g Ethylenglykol, 1720 N1 Luft und 230 N1 Stickstoff durch den Rohrreaktor. Zum Synthesegas wurden bei 24 % der Gesamthöhe der Katalysatorschüttung nach dem Hot Spot, der bei 15 % der Gesamthöhe der Schüttung lag, 1 ppm P, bezogen auf die eingesetzte Gewichtsmenge an Ethylenglykol, in Form von Triethylphosphat gegeben. Die Reaktortemperatur wurde mit Hilfe einer Salzschmelze auf 365°C eingestellt.

Die Gesamtgasmenge, bestehend aus Kreisgas und Synthesegas, betrug 9150 N1/h. Die GHSV (gas hourly space velocity), die definiert ist als

GHSV = Gasvolumen / Katalysatorvolumen

betrug 1610 $h^{-1}$. Die LHSV (liquid hourly space velocity), die definiert ist als

LHSV = Flüssigkeitsvolumen / Katalysatorvolumen

betrug 0,13 $h^{-1}$. Die Verweilzeit, definiert als Quotient aus dem Katalysatorvolumen und der Gasmenge war 2,3 s.

Das Reaktionsgas wurde nach Verlassen des Reaktors mit Wasser in Berührung gebracht, wobei die Reaktionsprodukte in der wässrigen Phase gelöst wurden. Die bei der Reaktion entstandenen Permanentgase CO und $CO_2$ verblieben im Abgas und wurden in der Gasphase analysiert.

Nach einer Laufzeit von 10 Tagen erhielt man eine Glyoxalausbeute von 74,4 Mol-%, bezogen auf eingesetztes Ethylenglykol, bei einem Ethylenglykolumsatz von 96,8 Mol-%. Die Verbrennung zu CO und $CO_2$ betrug 13 Mol-%. Als weitere Nebenprodukte entstanden 1,6 Mol-% Glyoxaldehyd und 4,8 Mol-% Formaldehyd.

Beispiel 4

7,2 kg Kupferformkörper wurden in einem Rohrreaktor aus rostfreiem Stahl mit einem inneren Durchmesser von 55 mm so eingebaut, daß die Katalysatorfüllhöhe 250 cm beträgt (Katalysatorvolumen: 5,7 1). Man leitete pro Stunde ein Synthesegasgemisch bestehend aus 840 g Ethylenglykol, 1800 N1 Luft und 150 N1 Stickstoff durch den Rohrreaktor. Zum Synthesegas wurden bei 24 % der Gesamthöhe der Katalysatorschüttung nach dem Hot Spot, der bei 15 % der Gesamthöhe der Schüttung lag, 1 ppm P, bezogen auf die eingesetzte Gewichtsmenge an Ethylenglykol, in Form von Triethylphosphat gegeben. Die Reaktortemperatur wurde mit Hilfe einer Salzschmelze auf 365°C eingestellt.

Die Gesamtgasmenge, bestehend aus Kreisgas und Synthesegas, betrug 9150 N1/h. Die GHSV (gas hourly space velocity), die definiert ist als

GHSV = Gasvolumen / Katalysatorvolumen

betrug 1610 $h^{-1}$. Die LHSV (liquid hourly space velocity), die definiert ist als

LHSV = Flüssigkeitsvolumen / Katalysatorvolumen

betrug 0,13 $h^{-1}$. Die Verweilzeit, definiert als Quotient aus dem Katalysatorvolumen und der Gasmenge war 2,3 s.

Das Reaktionsgas wurde nach Verlassen des Reaktors mit Wasser in Berührung gebracht, wobei die Reaktionsprodukte in der wässrigen Phase gelöst wurden. Die bei der Reaktion entstandenen Permanentgase CO und $CO_2$ verblieben im Abgas und wurden in der Gasphase analysiert.

Nach einer Laufzeit von 10 Tagen erhielt man eine Glyoxalausbeute von 75,8 Mol-%, bezogen auf eingesetztes Ethylenglykol, bei einem Ethylenglykolumsatz von 99,1 Mol-%. Die Verbrennung zu CO und $CO_2$ betrug 15,3 Mol-%. Als weitere Nebenprodukte entstanden 0,8 Mol-% Glyoxaldehyd und 4,7 Mol-% Formaldehyd.

## Patentansprüche

1. Verfahren zur Herstellung von Carbonylverbindungen der Formel

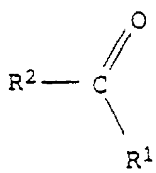

I ,

in der $R^1$ ein Wasserstoffatom oder einen Alkylrest mit 1 bis 3 C-Atomen, $R^2$ ein Wasserstoffatom oder einen Rest der Formel

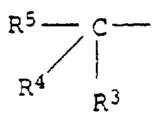

II ,

in der $R^3$ ein Wasserstoffatom oder zusammen mit $R^4$ ein Sauerstoffatom, $R^4$ den Rest $OR^6$ oder zusammen mit $R^3$ ein Sauerstoffatom, $R^5$ ein Wasserstoffatom, einen Alkylrest mit 1 bis 8 C-Atomen oder einen Cyclohexyl- oder Cyclopentylrest und $R^6$ einen Alkylrest mit 1 bis 4 C-Atomen, einen Cyclohexyl- oder Cyclopentylrest oder einen Rest der Formel $-CH_2-CHO$ oder $-CH_2-CH_2-O-CH_2-CHO$ bedeuten, durch Gasphasenoxidation von Methanol oder Alkoholen der Formel

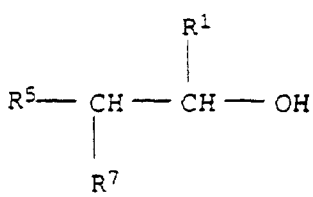

III ,

in der $R^1$ und $R^5$ die oben angegebene Bedeutung haben und $R^7$ ein Wasserstoffatom oder einen Rest $OR^8$ und $R^8$ ein Wasserstoffatom, einen Alkylrest mit 1 bis 4 C-Atomen, einen Cyclohexyl- oder Cyclopentylrest oder einen Rest der Formel $-CH_2-CH_2-OH$ oder $-CH_2-CH_2-O-CH_2-CH_2-OH$ bedeutet, mit einem Sauerstoff enthaltenden Gas in Gegenwart von Kupfer und/oder Silber enthaltenden Katalysatoren und einer unter Reaktionsbedingungen flüchtigen Phosphorverbindung in einer Menge, so daß die Phosphormenge (berechnet als P), bezogen auf die Gewichtsmenge an eingesetztem Alkohol, bis zu 20 ppm beträgt, **dadurch gekennzeichnet, daß** die Phosphormenge innerhalb der Katalysatorschüttung zugegeben wird.

2. Verfahren nach Anspruch 1, **dadurch gekennzeichnet, daß** die Phosphormenge in die Katalysatorschüttung im Bereich der oberen 0,1 bis 50 % der Gesamthöhe der Katalysatorschüttung zugegeben wird.

3. Verfahren nach Anspruch 1, **dadurch gekennzeichnet, daß** die Phosphormenge im Bereich der oberen 1 bis 35 % der Gesamthöhe der Katalysatorschüttung zugegeben wird.

4. Verfahren nach Anspruch 1, **dadurch gekennzeichnet, daß** die Phosphormenge (berechnet als P), bezogen auf die Gewichtsmenge an dem eingesetzten Alkohol, 0,05 bis 20 ppm beträgt.

5. Verfahren nach Anspruch 1, **dadurch gekennzeichnet, daß** die Phosphormenge in mindestens zwei Anteilen zugegeben wird.

6. Verfahren nach Anspruch 5, **dadurch gekennzeichnet, daß** das Gewichtsverhältnis des ersten Anteils der Phosphormenge zu dem mindestens einen weiteren Anteil 0,005 bis 200 beträgt.

7. Verfahren nach Anspruch 1, **dadurch gekennzeichnet, daß** man Glyoxal aus Ethylenglykol herstellt.

**Claims**

1. A process for preparing carbonyl compounds of the formula

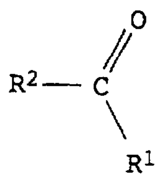

I ,

where $R^1$ is a hydrogen atom or an alkyl radical having from 1 to 3 carbon atoms, $R^2$ is a hydrogen atom or a radical of the formula

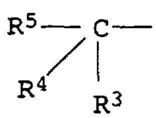

II ,

where $R^3$ is a hydrogen atom or together with $R^4$ is an oxygen atom, $R^4$ is the radical $OR^6$ or together with $R^3$ is an oxygen atom, $R^5$ is a hydrogen atom, an alkyl radical having from 1 to 8 carbon atoms or a cyclohexyl or cyclopentyl radical and $R^6$ is an alkyl radical having from 1 to 4 carbon atoms, a cyclohexyl or cyclopentyl radical or a radical of the formula $-CH_2-CHO$ or $-CH_2-CH_2-O-CH_2-CHO$, by gas-phase oxidation of methanol or alcohols of the formula

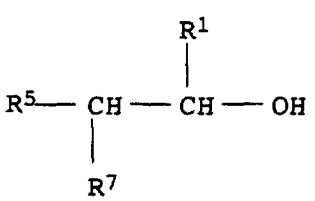

III ,

where $R^1$ and $R^5$ are as defined above and $R^7$ is a hydrogen atom or a radical $OR^8$ and $R^8$ is a hydrogen atom, an alkyl radical having from 1 to 4 carbon atoms, a cyclohexyl or cyclopentyl radical or a radical of the formula $-CH_2-CH_2-OH$ or $-CH_2-CH_2-O-CH_2-CH_2-OH$, using an oxygen-containing gas in the presence of copper- and/or silver-containing catalysts and an amount of a phosphorus compound which is volatile under the reaction conditions which is such that the amount of phosphorus (calculated as P) is up to 20 ppm, based on the weight of alcohol

used, wherein the phosphorus is introduced within the catalyst bed.

2. A process as claimed in claim 1, wherein the phosphorus is introduced into the catalyst bed in the region of the upper 0.1-50 % of the total height of the catalyst bed.

3. A process as claimed in claim 1, wherein the phosphorus is introduced in the region of the upper 1-35 % of the total height of the catalyst bed.

4. A process as claimed in claim 1, wherein the amount of phosphorus (calculated as P) is from 0.05 to 20 ppm, based on the weight of the alcohol used.

5. A process as claimed in claim 1, wherein the phosphorus is introduced in at least two portions.

6. A process as claimed in claim 5, wherein the weight ratio of the first portion of phosphorus to the further portion or portions is from 0.005 to 200.

7. A process as claimed in claim 1, wherein glyoxal is prepared from ethylene glycol.

## Revendications

1. Procédé de préparation de composés carbonyle de formule:

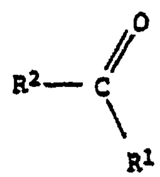

I ,

dans laquelle $R^1$ représente un atome d'hydrogène ou un résidu alkyle ayant 1 à 3 atomes de C, $R^2$ représente un atome d'hydrogène ou un résidu de formule

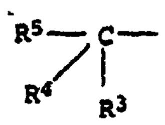

II ,

dans laquelle, $R^3$ représente un atome d'hydrogène ou ensemble avec $R^4$, un atome d'oxygène, $R^4$ représente le résidu $OR^6$ ou ensemble avec $R^3$ un atome d'oxygène, $R^5$ représente un atome d'hydrogène, un résidu alkyle ayant 1 à 8 atomes de C, ou un résidu cyclohexyle ou cyclopentyle et $R^6$ représente un résidu alkyle ayant 1 à 4 atomes de C, un résidu cyclohexyle ou cyclopentyle ou un résidu de formule $-CH_2-CHO$ ou $-CH_2-CH_2-O-CH_2-CHO$, au moyen d'une oxydation en phase gazeuse du méthanol ou d'alcools de formule

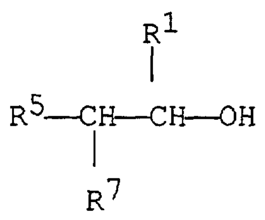

III,

dans laquelle $R^1$ et $R^5$ prennent la signification indiquée ci-dessus et $R^7$ représente un atome d'hydrogène

ou un résidu $OR^8$ et $R^8$ un atome d'hydrogène, un résidu alkyle ayant 1 à 4 atomes de C, un résidu cyclohexyle ou cyclopentyle ou un résidu de formule $-CH_2-CH_2-HO$ ou $-CH_2-CH_2-O-CH_2-CH_2-HO$, avec un gaz contenant de l'oxygène en présence de catalyseurs contenant du cuivre et/ou de l'argent et un composé du phosphore volatil dans les conditions de la réaction, en une quantité telle que la quantité de phosphore (calculée en P) est de jusqu'à 20 ppm, par rapport à la quantité pondérale de l'alcool mis en oeuvre, **caractérisé en ce que** la quantité de phosphore est ajoutée à l'intérieur du catalyseur en vrac.

**2.** Procédé selon la revendication 1, **caractérisé en ce que** la quantité de phosphore dans le catalyseur en vrac est ajoutée dans la zone supérieure correspondant aux 0,1 à 50% de la hauteur totale du catalyseur en vrac.

**3.** Procédé selon la revendication 1, **caractérisé en ce que** la quantité de phosphore dans le catalyseur en vrac est ajoutée dans la zone supérieure correspondant aux 0,1 à 35% de la hauteur totale du catalyseur en vrac.

**4.** Procédé selon la revendication 1, **caractérisé en ce que** la quantité de phosphore (calculée en P) est de 0,05 à 20 ppm par rapport à la quantité pondérale de l'alcool mis en oeuvre.

**5.** Procédé selon la revendication 1, **caractérisé en ce que** la quantité de phosphore est ajoutée au moins en deux portions.

**6.** Procédé selon la revendication 5, **caractérisé en ce que** le rapport pondéral de la première portion de la quantité de phosphore est de 0,005 à 200 par rapport à ladite au moins une portion supplémentaire.

**7.** Procédé selon la revendication 1, **caractérisé en ce que** l'on prépare du glyoxal à partir de l'éthylèneglycol.